# EUROPEAN PATENT SPECIFICATION

(11) **EP 3 842 000 B1**
(45) Date of publication and mention of the grant of the patent: **07.10.2026**
(21) Application number: 20217038.7
(22) Date of filing: 23.12.2020
(51) Int. Cl.: A61B 18/14, A61B 5/00, A61B 18/00, A61B 34/20, A61B 90/00, A61B 5/06

(54) **CONTACT FORCE SPRING WITH MECHANICAL STOPS**
KONTAKTKRAFTFEDER MIT MECHANISCHEN ANSCHLÄGEN
RESSORT DE FORCE DE CONTACT COMPORTANT DES BUTÉES MÉCANIQUES

(30) Priority: 24.12.2019 US 201916726605
(43) Date of publication of application: 30.06.2021
(73) Proprietor: Biosense Webster (Israel) Ltd., Yokneam 2066717 (IL)
(72) Inventor: BEECKLER, Christopher Thomas, Irvine, CA 92618 (US); KEYES, Joseph Thomas, Irvine, CA 92618 (US)
(74) Representative: Carpmaels & Ransford LLP

(56) References cited:
- EP-A1- 3 189 806
- US-A1- 2008 294 158
- US-A1- 2013 253 503
- US-A1- 2018 271 590
- US-A1- 2018 338 811

## Description

### FIELD OF THE INVENTION

The present invention relates to medical equipment, and in particular, but not exclusively to, contact force springs.

### BACKGROUND

In some diagnostic and therapeutic techniques, a catheter is inserted into a chamber of the heart and brought into contact with the inner heart wall. In such procedures, it is generally important that the distal tip of the catheter engages the endocardium with sufficient pressure to ensure good contact. Excessive pressure, however, may cause undesired damage to the heart tissue and even perforation of the heart wall.

For example, in intracardiac radio-frequency (RF) ablation, a catheter having an electrode at its distal tip is inserted through the patient's vascular system into a chamber of the heart. The electrode is brought into contact with a site (or sites) on the endocardium, and RF energy is applied through the catheter to the electrode in order to ablate the heart tissue at the site. Proper contact between the electrode and the endocardium during ablation is necessary in order to achieve the desired therapeutic effect without excessive damage to the tissue.

US Patent Publication 2011/0263934 of Aeby, et al., describes a catheter for diagnosis or treatment of a vessel or organ is provided in which a flexible elongated body includes a tri-axial force sensor formed of a housing and a plurality of optical fibers associated with the housing that measure changes in the intensity of light reflected from the lateral surfaces of the housing resulting from deformation caused by forces applied to a distal extremity of the housing. A controller receives an output of the optical fibers and computes a multi-dimensional force vector corresponding to the contact force.

US Patent Publication 2011/0130648 of Beeckler, et al., describes a medical probe, consisting of a flexible insertion tube, having a distal end for insertion into a body cavity of a patient, and a distal tip, which is disposed at the distal end of the flexible insertion tube is configured to be brought into contact with tissue in the body cavity. The probe also includes a coupling member, which couples the distal tip to the distal end of the insertion tube and which consists of a tubular piece of an elastic material having a plurality of intertwined helical cuts therethrough along a portion of a length of the piece.

US Patent Publication 2016/0339207 of Beeckler, et al., describes a catheter having a catheter shaft that has a more uniform construction throughout its length and is able to provide more than one deflection curvature. The catheter shaft includes a flexible outer tubular member, and a less flexible inner tubular member extending through the outer tubular member in a proximal section of the catheter shaft, wherein the inner tubular member is afforded longitudinal movement relative to the outer tubular member. The catheter also includes at least one puller wire extending through the inner tubular member to deflect a distal deflection section of the catheter shaft, wherein longitudinal movement of the inner tubular member relative to the outer tubular member enables an operator to select and set a deflection curvature of the distal deflection section.

US2018338811A1 relates to a spring element for a contact force sensing medical catheter. The spring element includes a first ring, a second ring spaced apart from the first ring, a plurality of struts connecting the first ring to the second ring, and a plurality of interlocking features. The second ring and the first ring are coaxially aligned with a longitudinal axis of the spring element. The plurality of struts is configured to permit elastic, relative axial and radial displacement between the first ring and the second ring. The plurality of interlocking features is configured to limit the relative axial and radial displacement between the first ring and the second ring.

### SUMMARY

There is provided in accordance with the present invention a catheter apparatus according to claim 1. Further embodiments of the invention are described by the dependent claims.

### BRIEF DESCRIPTION OF THE DRAWINGS

The present invention will be understood from the following detailed description, taken in conjunction with the drawings in which:
Fig. 1 is a schematic view of a catheter constructed and operative in accordance with an embodiment of the present disclosure;
Figs 2A-B are schematic views of the catheter of Fig. 1 with an outer sleeve removed;
Fig. 3 is a schematic view of the catheter of Fig. 1 with several elements removed;
Fig. 4 is a partially exploded view of the catheter as shown in Fig. 3;
Figs. 5A-C are schematic views of a proximal coupler of the catheter of Fig. 1;
Figs. 6A-B are schematic views of the distal coupler of the catheter of Fig. 1;
Figs. 7A-B are schematic views of a spring of the catheter of Fig. 1;
Figs. 8A-C are schematic views of a force sensor of the catheter of Fig. 1; and
Fig. 9 is a schematic view of a spring constructed and operative in accordance with an embodiment of the present invention

### DESCRIPTION OF EXAMPLE EMBODIMENTS OVERVIEW

An assembly, such as a balloon, at the tip of a contact force sensor of a catheter presents a unique challenge to a contact force spring of the force sensor. The spring experiences significantly higher tensile forces as the assembly is withdrawn into the catheter sheath, or other such maneuvers. In addition to the withdrawal forces, even during normal use side and other forces exerted on the spring are higher than those exerted with a focal catheter due to the length of the assembly, e.g., a balloon.

For example, the contact force spring may be formed from one or more helices cut around a tube. The spring functions very well when attached to a focal catheter. However, in the case of other catheters such as a balloon catheter, the higher forces on the spring, typically when the balloon is withdrawn into its sheath, or pulling the balloon on a Haemostatic valve, can permanently damage the spring, for example, the ends of the helical cuts may open too much.

Embodiments of the present invention solve the above problems by adding deviations in the helical cut(s) in a spring to provide mechanical stops which prevent overbending and overstretching of the spring. The mechanical stops are designed to engage once the spring has been extended by a preset amount, and the engagement prevents plastic deformation, which is irreversible, of the spring. The mechanical stops are generally designed to prevent overstretching while still allowing for compression of the spring so that the spring can still perform its main function in measuring force. Alternate shapes of mechanical stops are possible.

**In** some embodiments, the mechanical stops are designed to engage simultaneously rather than sequentially in order to share the load evenly, otherwise one (or more) mechanical stop(s) will take the entire load until it (or they) fails, at which point the next mechanical stop(s) would take the load, etc.

In some embodiments, a catheter includes an elongated deflectable element, a distal assembly (which may include an expandable distal assembly, e.g., including an inflatable balloon), a force sensor disposed between the elongated deflectable element and the distal assembly. The force sensor includes a tube with helical cuts extending around a circumference of the tube. Each helical cut includes deviations extending in a longitudinal direction of the tube. The deviations prevent overstretching and overbending of the spring. The force sensor may include position coils disposed on the tube.

The tube may include any suitable number of helical cuts, for example, two, three, or more than three. The term "helical cut", as used in the specification and claims, is defined as a helical cut extending more than one turn around the tube, or extending at least half of a turn around the tube.

Respective deviations of respective helical cuts define respective mechanical stops. Each mechanical stop includes opposing surfaces which come into contact with each other to prevent overstretching and overbending of the spring.

In some embodiments, respective mechanical stops are designed to engage simultaneously so that a force applied on the spring is shared among the respective mechanical stops to prevent sequential failure of the respective mechanical stops. In some embodiments, at least two mechanical stops of each helical cut are designed to engage simultaneously. In some embodiments, all the mechanical stops of each helical cut are designed to engage simultaneously.

In some embodiment the deviations include respective opposing sigmoid curves. In some embodiments, respective mechanical stops include respective loops and sockets. In some embodiments, respective mechanical stops include respective T-shape elements placed in respective T-shape openings. According to the invention, respective mechanical stops include respective L-shape elements placed in respective L- shape openings.

In some embodiments, the catheter includes a proximal coupler having a proximal end connected to the distal end of the elongated deflectable element. The tube and the distal end of the proximal coupler include complementary bayonet connecting features connecting the distal end of the proximal coupler with the tube.

In some embodiments, the catheter includes a distal coupler and the distal end of the tube includes holes around its circumference. The distal end of the tube is connected to the distal coupler via an adhesive (such as epoxy) which extends into the holes to promote adhesion between the distal coupler and the tube.

In some embodiments, the distal edge of the tube includes openings (e.g., U-shape openings) for engaging protrusions of the distal coupler to prevent rotation of the distal coupler with respect to the tube.

### SYSTEM DESCRIPTION

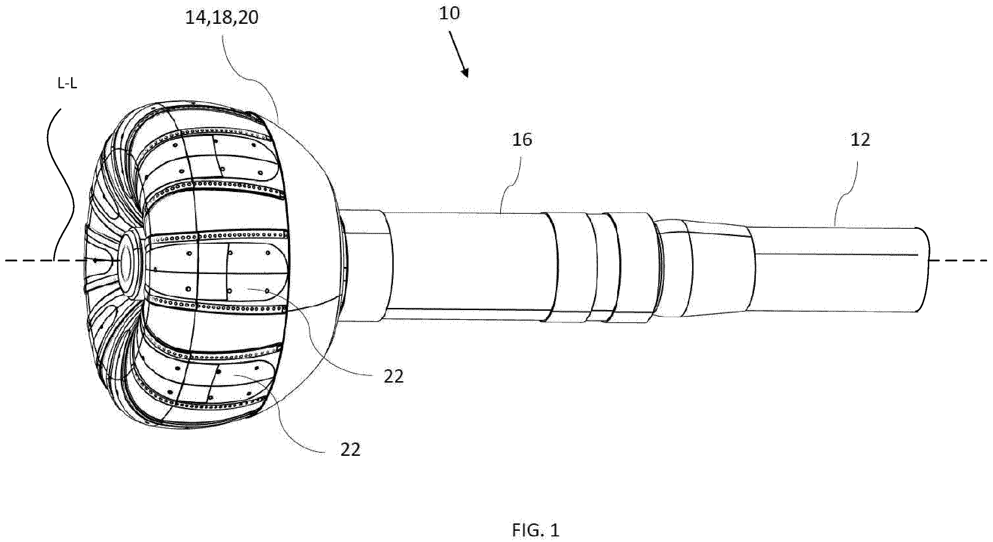

Reference is now made to Fig. 1, which is a schematic view of a catheter 10 constructed and operative in accordance with an embodiment of the present invention. The catheter 10 includes an elongated deflectable element 12, a distal assembly 14 and an outer sleeve 16 disposed about a longitudinal axis L-L (which will be used to reference various internal and external components of catheter 10). The distal assembly 14 may include any suitable distal assembly, for example, a lasso catheter assembly or a focal catheter assembly. In some embodiments, the distal assembly 14 includes an expandable distal assembly 18, which may include an inflatable balloon 20 or a basket, by way of example only. The elongated deflectable element 12 includes lumens (not shown) in which to carry electrical connections, irrigation channels, puller wires, and the like. The distal assembly 14 may also include multiple electrodes 22 (only two labeled for the sake of simplicity) disposed thereon for use in mapping and/or ablation or any other suitable function.

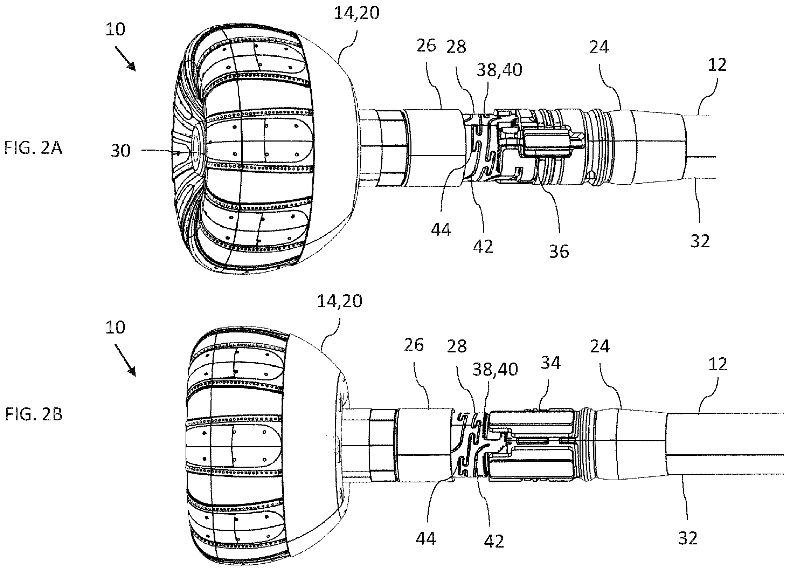

Reference is now made to Figs 2A-B, which are schematic views of different sides of the catheter 10 of Fig. 1 with the outer sleeve 16 of Fig. 1 removed. The catheter 10 includes a proximal coupler 24, a distal coupler 26, and a force sensor 28.

The force sensor 28 is disposed between the elongated deflectable element 12 and the distal assembly 14, and more specifically disposed between the proximal coupler 24 and the distal coupler 26.

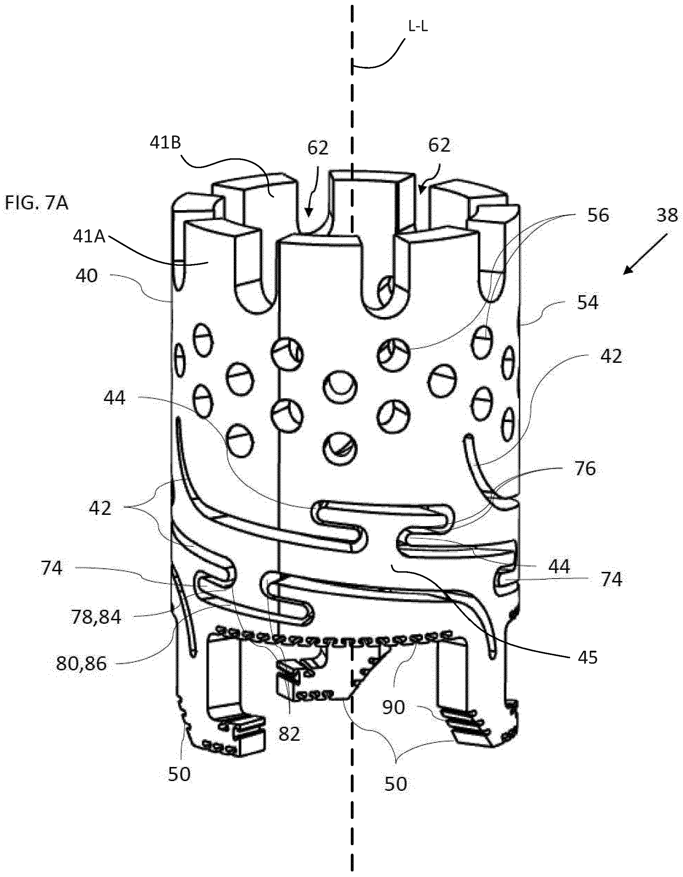

The force sensor 28 includes a spring 38 including a tube 40 with one or more helical cuts 42 extending around a circumference of the tube 40. As used herein, the circumference includes both the outer circumferential surface 41A and the inner circumferential surface 41B of the tubular member 40. Each helical cut 42 includes deviations 44 extending in a longitudinal direction of the tube 40. The deviations 44 are configured to prevent overstretching and overbending of the spring 38. The spring 38 is described in more detail with reference to Figs. 7A-8C.

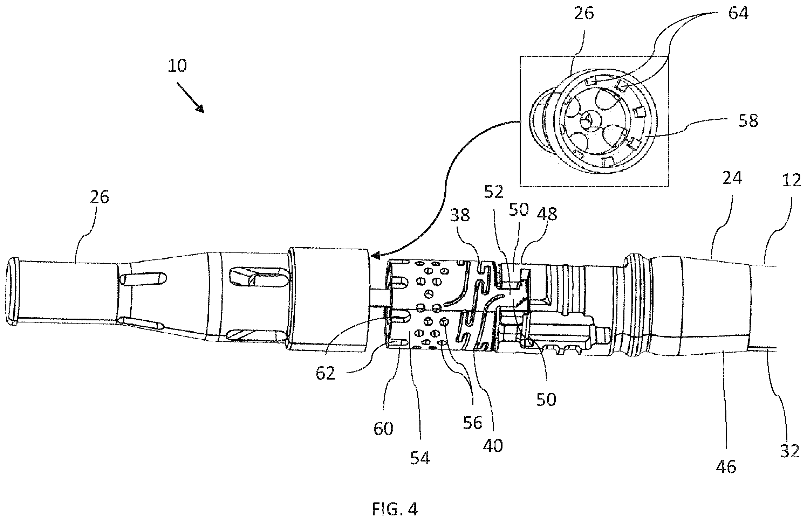

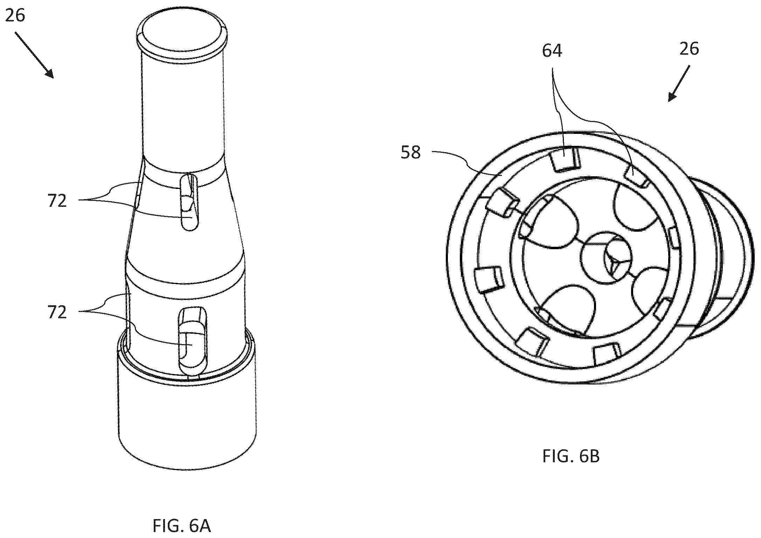

The inflatable balloon 20 is mounted on the distal coupler 26 with the distal coupler 26 extending until a nose 30 in the center of the distal end of the inflatable balloon 20. Therefore, the distal coupler 26 couples the inflatable balloon 20 with the force sensor 28. The distal coupler 26 is described in more detail with reference to Figs. 4, 6A-B.

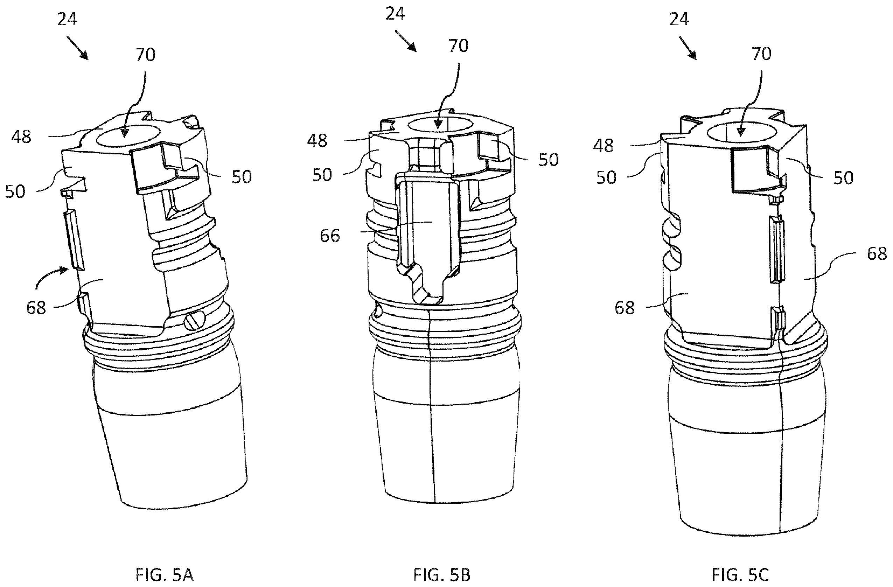

The proximal coupler 24 couples the force sensor 28 with a distal end 32 of the elongated deflectable element 12. The catheter 10 includes a position sensor 34 (Fig. 2B), such as a single, dual, and/or triple-axis coil. The position sensor 34 is mounted on the proximal coupler 24 in the examples of Fig. 2B. The catheter 10 also includes a solder pad 36 (Fig. 2A) disposed on the proximal coupler 24 to which various electrical connections from components at the distal end of the catheter 10 and electrical connections from the proximal end of the catheter 10 may be connected. The proximal coupler 24 is described in more detail with reference to Figs. 5A-C.

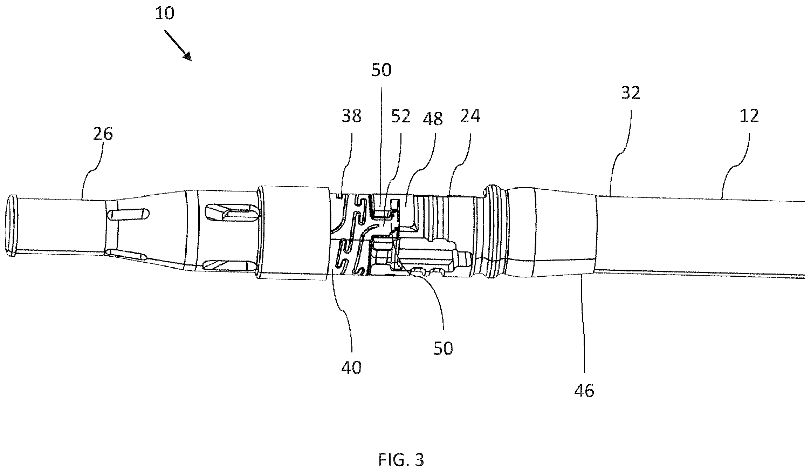

Reference is now made to Figs. 3 and 4. Fig. 3 is a schematic view of the catheter 10 of Fig. 1 with several elements removed. Fig. 3 shows the catheter 10 with the distal assembly 14 and the sleeve 16 of Fig. 1 removed, and with the solder pad 36 and the position sensor 34 of Figs. 2A and 2B removed. Fig. 4 is a partially exploded view of the catheter 10 as shown in Fig. 3. Fig. 4 shows the distal coupler 26 pulled away from the spring 38 to show how the distal coupler 26 and the spring 38 are connected to each other.

The distal end 32 of the elongated deflectable element 12 is connected to a proximal end 46 of the proximal coupler 24. A proximal end 52 of the tube 40 and a distal end 48 of the proximal coupler 24 include complementary bayonet connecting features 50 connecting the distal end 48 of the proximal coupler with the proximal end 52 of the tube 40. The bayonet connecting features 50 are described in more detail with reference to Figs. 5A-C and Figs. 7A-B.

Fig. 4 shows that a distal end 54 of the tube 40 comprises holes 56 (only some labeled for the sake of simplicity) disposed around the circumference of the tube 40. The distal end 54 of the tube 40 is connected to the distal coupler 26 distal coupler via an adhesive, e.g., epoxy, which extends into respective ones of the holes 56 thereby improving a bond between the distal end 54 of the tube 40 and an inner proximal surface 58 of the distal coupler 26. The holes 56 are described in more detail with reference to Figs. 7A-B.

A distal edge 60 of the tube 40 includes openings 62 (only some labeled for the sake of simplicity) disposed around the circumference of the distal edge 60. The inner proximal surface 58 of the distal coupler 26 includes protrusions 64 (only some labeled for the sake of simplicity), disposed circumferentially around the inner proximal surface 58, and configured for engaging the openings 62 (or slots disposed around the perimeter of the generally tubular member 38) to prevent rotation of the distal coupler 26 with respect to the tube 40. The openings 62 and the protrusions 64 may be any suitable shape. In some embodiments, the openings 62 include U-shaped openings as shown in the example of Fig. 4 or rectangular shape openings. The openings 62 and the protrusions 64 are described in more detail with reference to Fig. 6B and Figs. 7A-B.

Reference is now made to Figs. 5A-C, which are schematic views of various sides of the proximal coupler 24 of the catheter 10 of Fig. 1. The proximal coupler 24 has a generally cylindrical shape with a central lumen 70 for passing various elements therein such as wires and an irrigation tube (not shown). The distal end 48 includes the bayonet connecting features 50 which may have any suitable shape, for example, but not limited to an L-shape recess. In some embodiments the distal end 48 includes three bayonet connecting features 50. However, the distal end 48 may include more or less than three bayonet connecting features 50. The proximal coupler 24 includes a recess 66 (Fig. 5B) for accepting the solder pad 36 (Fig. 2A) therein in addition to allowing a passage for wires connected to solder pad 36 to pass into coupler 24. Coupler 24 also includes two flat surfaces 68 (Figs. 5A and 5C) for accepting the position sensor 34 (Fig. 2B) thereon. The proximal coupler 24 may be formed from any suitable material or combination of materials, for example, but not limited to, polycarbonate with or without glass filler, polyether ether ketone (PEEK) with or without glass filler, or polyetherimide (PEI) with or without glass filler. The proximal coupler 24 may have any suitable outer diameter, for example, in a range of 1 mm to 10 mm.

Reference is now made to Figs. 6A-B, which are schematic views of the distal coupler 26 of the catheter 10 of Fig. 1. Fig. 6A shows that the distal coupler 26 includes openings 72 for irrigation and/or feeding wires to the electrodes 22 (Fig. 1) of the distal assembly 14 (Fig. 1). In some embodiments, the openings 72 may be disposed around a circumference of the distal coupler 26 in a proximal portion of the distal coupler 26 and/or in a distal portion of the distal coupler 26. The distal coupler 26 may include any suitable number of openings 72, for example, in a range between 1 and 12 openings 72, such as 8 openings.

Fig. 6B shows the inner proximal surface 58 and its protrusions 64 (only some labeled for the sake of simplicity). The distal coupler 26 may include any suitable number of protrusions 64 to engage with the openings 62 (Fig. 4) of the tube 40 (Fig. 4). In some embodiments, the distal coupler 26 includes eight protrusions 64, but may be in the range of 1 to 30 protrusions 64, by way of example. The protrusions 64 may have any suitable dimensions, in the order of 0.05 mm to 10 mm and may depend on the number of protrusions 64. The distal coupler 26 may be formed from any suitable material or combination of materials, for example, but not limited to, polycarbonate with or without glass filler, polyether ether ketone (PEEK) with or without glass filler, or polyetherimide (PEI) with or without glass filler. The distal coupler 26 may have any suitable outer diameter, for example, in a range of 1 mm to 10 mm.

Reference is now made to Figs. 7A-B, which are schematic views of the spring 38 of the catheter 10 of Fig. 1. As previously mentioned, the spring 38 includes the tube 40 with helical cuts 42 extending around a circumference of the tube 40. As seen in Fig. 7A, two generally parallel helical cuts extend through the tubular member 38 while traversing along the longitudinal axis L-L to define a helical shaped spring member 45 (between the gaps 42) which is separated from the tubular member 38 by the gaps or cuts 42. As shown in Fig. 7A, three helical spring members 45 are provided. The helical cuts 42 provide the coupler 38 with its ability to bend and compress while provide a resistance to bending and compression. Each helical cut 42 includes deviations 44 extending in a longitudinal direction (approximately parallel to the axis L-L) of the tube 40. The deviations 44 are configured to prevent overstretching and overbending of the spring 45 by having a portion of the spring 45 configured to interlock with another portion of the tubular member 38 via a suitable interlock 44.

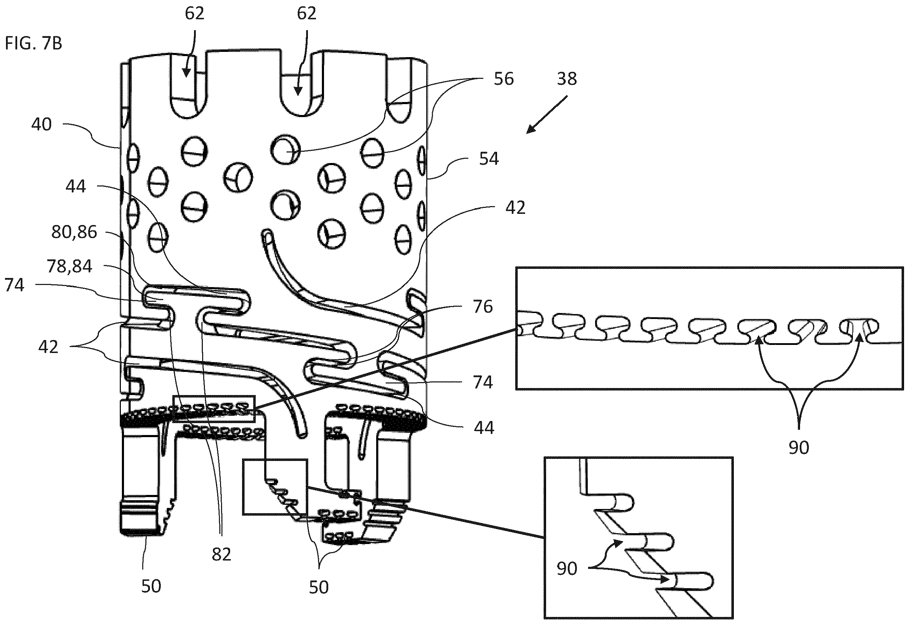

The helical cuts 42 extend from the outer surface of the tube 40 through to the inner surface of the tube 40. The helical cuts 42 may be right-handed helices or left-handed helices. The pitch of each helical cut 42 may be any suitable value, for example in the range of 0.2 mm to 5 mm. The helical cuts 42 may have any suitable width, for example, in the range of 0.05 mm to 0.5 mm, such as 0.1 mm. The spring 38 may include any suitable number of helical cuts 42, for example, two helical cuts 42 forming a double helix, or three helical cuts 42 forming a triple helix, and so on. The helical cuts 42 are generally circular helices disposed about a longitudinal axis L-L extending through the center of member 38. Each of the helical cuts 42 may extend around the circumference for any suitable amount of turns, including extending for less than one turn but at least half a turn. For example, any of the helical cuts 42 may extend 1.5 turns, 1 turn, two-thirds of a turn (as shown in Figs. 7A and 7B), or half a turn. In the example of Figs. 7A-B, respective ones of the helical cuts 42 extend from respective ones of the bayonet connecting features 50 up and around the circumference of the tube 40.

Respective ones of the deviations 44 of respective ones of the helical cuts 42 define respective mechanical stops 74 (only some labeled for the sake of simplicity). Each mechanical stop 74 includes opposing surfaces 76 (only some labeled for the sake of simplicity) which are configured to come into contact with each other to prevent overstretching and overbending of the spring 38. In some embodiments, the opposing surfaces 76 of respective ones of the mechanical stops 74 are configured to engage simultaneously so that a force applied on the spring 38 is shared among the respective mechanical stops 74 to prevent sequential failure of the respective mechanical stops 74. In some embodiments, at least two, and generally all, of the mechanical stops 74 of each helical cut 42 are configured to engage simultaneously. In some embodiments, respective ones of the mechanical stops 74 include respective loops 78 and sockets 80 (only one labeled for the sake of simplicity).

In some embodiments, respective one of the deviations 44 include respective opposing sigmoid curves 82 (only two labeled for the sake of simplicity) with characteristic S-shapes so that the deviations 44, and the mechanical stops 74, are formed by an S-shape and a reversed S-shape as shown in Figs. 7A-B. In some embodiments, respective ones of the mechanical stops 74 include respective T-shape elements 84 disposed in respective T-shape openings 86.

The bayonet connecting features 50 of the tube 40 are generally L-shaped and are configured to connect with the bayonet connecting features 50 of the proximal coupler 24 (Figs, 5A-C). The tube 40 may include any suitable number of bayonet connecting features 50, for example, one, two, three or more.

The holes 56 (only some labeled for the sake of simplicity) in the distal end 54 of the tube 40 are disposed around the circumference of the tube 40. The tube 40 may include any suitable number of holes 56, for example, two or more. The maximum number of holes 56 is generally restricted by the available space on the tube 40 above the helical cuts 42. Each hole 56 may have any suitable diameter, for example, in the range of 0.05 mm to 0.5 mm.

In some embodiments, the tube 40 includes eight openings 62 (only some labeled for the sake of simplicity), but may have any suitable number of openings 62, for example, in the range of 1 to 30 openings 62. The openings 62 may have any suitable dimensions, in the order of 0.05 mm to 10 mm and may depend on the number of openings 62.

The lower surface of the tube 40 may include notches 90 (only some labeled for the sake of simplicity) to enhance adhesive connection between the lower surface of the tube 40 and other elements of the catheter 10, such as proximal coupler 24.

The helical cuts 42, holes 56, and openings 62 may be formed by any suitable method, for example, by laser machining, electric discharge machining, or conventional machining. The tube 40 may have any suitable outer diameter, for example, in the range of 1 mm to 10 mm. The tube 40 may have any suitable wall thickness, for example, in the range of 0.1 mm to 3 mm. The tube may be formed from any suitable material, for example, Nitinol, beta titanium, beryllium copper, or phosphor bronze.

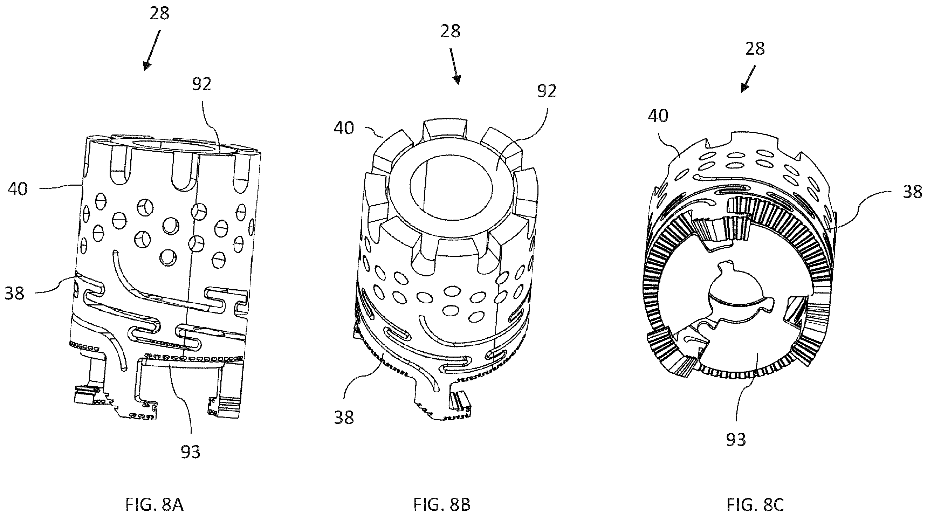

Reference is now made to Figs. 8A-C, which are schematic views of the force sensor 28 of the catheter 10 of Fig. 1. The force sensor 28 includes the spring 38 and a transmitting coil 92 disposed on the top and receiving coils 93 disposed on the bottom of the tube 40. The receiving coils 93 receive a transmitted signal from the transmitting coil 92. The received signal is representative of a distance between the transmitting coil 92 and the receiving coils 93 thereby providing an indication of a force applied to the force sensor 28. Any suitable transmitting coil 92, and receiving coils 93 may be used, for example, the coils described in US Patent Publication 2018/0256247 of Govari, et al. In some embodiments, the positioning of the transmitting coil 92 and the receiving coils 93 may be reversed or in any suitable position. In some embodiments, the force sensor 28 may include a single receiving coil and multiple transmitting coils, or any suitable combination thereof.

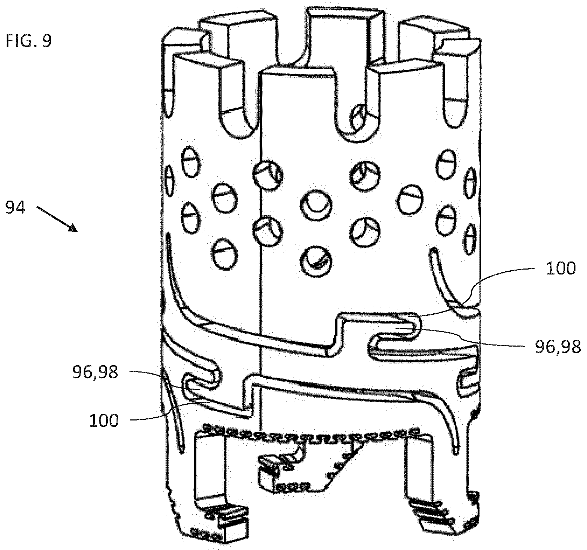

Reference is now made to Fig. 9, which is a schematic view of a spring 94 constructed and operative in accordance with an embodiment of the present invention. The spring 94 includes mechanical stops 96. Respective mechanical stops 96 include respective L-shape elements 98 disposed in respective L- shape openings 100.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 72% to 108%.

Various features of the invention which are, for clarity, described in the contexts of separate embodiments may also be provided in combination in a single embodiment. Conversely, various features of the invention which are, for brevity, described in the context of a single embodiment may also be provided separately or in any suitable sub-combination.

The embodiments described above are cited by way of example, and the present invention is not limited by what has been particularly shown and described hereinabove. The invention is defined by the following claims.

## Claims

1. A catheter apparatus (10), comprising:
an elongated deflectable element (12);
a distal assembly (14);
a force sensor (28) disposed between the elongated deflectable element and the distal assembly, and comprising a spring (38) including a tube (40) with at least one helical cut (42) extending around a circumference of the tube, the at least one helical cut including deviations (44) extending in a longitudinal direction of the tube, the deviations being configured to prevent overstretching and overbending of the spring;
wherein respective ones of the deviations of the helical cuts define respective mechanical stops (74, 96), each mechanical stop including opposing surfaces which are configured to come into contact with each other to prevent overstretching and overbending of the spring;
**characterised in that**
respective ones of the mechanical stops include respective L-shape elements (98) disposed in respective L-shape openings (100)

2. The apparatus according to claim 1, wherein respective ones of the deviations include respective opposing sigmoid curves (82).

3. The apparatus according to claim 1, wherein respective ones of the mechanical stops are configured to engage simultaneously so that a force applied on the spring is shared among the respective mechanical stops to prevent sequential failure of the respective mechanical stops.

4. The apparatus according to claim 3, wherein the at least one helical cut comprises multiple helical cuts (42), (i) respective ones of the mechanical stops of each of the helical cuts being configured to engage simultaneously, or (ii) the mechanical stops of each of the helical cuts being configured to engage simultaneously.

5. The apparatus according to claim 1, wherein the at least one helical cut comprises multiple helical cuts (42), the tube comprising three of the helical cuts.

6. The apparatus according to claim 1, further comprising a proximal coupler (24) having a proximal and distal end (46, 48), wherein the elongated deflectable element has a distal end (32) connected to the proximal end of the proximal coupler, the tube and the distal end of the proximal coupler including complementary bayonet connecting features (50) connecting the distal end of the proximal coupler with the tube.

7. The apparatus according to claim 1, further comprising a distal coupler (26), wherein the tube has a distal end (54) comprising holes (56) disposed around the circumference of the tube, the distal end of the tube being connected to the distal coupler via an adhesive which extends into respective ones of the holes.

8. The apparatus according to claim 7, wherein the tube includes a distal edge (60) with openings (62) therein, the distal coupler including protrusions (64) configured for engaging the openings to prevent rotation of the distal coupler with respect to the tube.

9. The apparatus according to claim 8, wherein the openings include U-shaped openings.

10. The apparatus according to claim 1, further comprising a distal coupler (26), wherein the tube includes a distal edge (60) with openings therein, the distal coupler including protrusions (54) configured for engaging the openings to prevent rotation of the distal coupler with respect to the tube, optionally wherein the openings include U-shaped openings.

11. The apparatus according to claim 1, wherein the distal assembly includes an expandable distal assembly (18), optionally wherein the expandable distal assembly includes an inflatable balloon (20).

12. The apparatus according to claim 1, wherein the force sensor includes a transmitting coil (92) and at least one receiving coil (93) disposed on the tube.

13. The catheter apparatus of claim 1,
wherein the tube extends along a longitudinal axis from a first end (54) to a second end (52) to define a circumferential surface;
wherein a plurality of slots (62) are disposed proximate the first end on the circumferential surface of the tubular member about the longitudinal axis, each slot being generally parallel to the longitudinal axis;
wherein a bayonet member (50) is disposed proximate the second end, the bayonet member includes three arms disposed on the circumference of the tubular member about the longitudinal axis;
wherein the at least one helical cut comprises at least two parallel cuts (42) extending through the surface of the generally tubular member for a portion of the circumference of the tubular member and along the longitudinal axis so that the parallel cuts define at least one helical shaped spring member disposed between the first end and the second end proximate the circumference of the tubular member; and
wherein the deviations define a mechanical stop (74, 96) so that the helical spring member is prevented from overbending or overstretching.

## Patentansprüche

1. Kathetereinrichtung (10), umfassend:
ein längliches ablenkbares Element (12);
eine distale Anordnung (14);
einen Kraftsensor (28), der zwischen dem länglichen ablenkbaren Element und der distalen Anordnung angeordnet ist und eine Feder (38) umfasst, die ein Rohr (40) mit mindestens einem spiralförmigen Schnitt (42) einschließt, der sich um einen Umfang des Rohrs erstreckt, wobei der mindestens eine spiralförmige Schnitt Abweichungen (44) einschließt, die sich in einer Längsrichtung des Rohrs erstrecken, wobei die Abweichungen konfiguriert sind, um ein Überdehnen und Überbiegen der Feder zu verhindern;
wobei jeweilige der Abweichungen der spiralförmigen Schnitte jeweilige mechanische Anschläge (74, 96) definieren, wobei jeder mechanische Anschlag gegenüberliegende Flächen einschließt, die konfiguriert sind, um miteinander in Kontakt zu kommen, um ein Überdehnen und Überbiegen der Feder zu verhindern;
**dadurch gekennzeichnet, dass** jeweilige der mechanischen Anschläge jeweilige L-förmige Elemente (98) einschließen, die in jeweiligen L-förmigen Öffnungen (100) angeordnet sind

2. Einrichtung nach Anspruch 1, wobei jeweilige der Abweichungen jeweilige entgegengesetzte Sigmoidkurven (82) einschließen.

3. Einrichtung nach Anspruch 1, wobei jeweilige der mechanischen Anschläge konfiguriert sind, um gleichzeitig einzugreifen, sodass eine auf die Feder ausgeübte Kraft unter den jeweiligen mechanischen Anschlägen aufgeteilt wird, um ein sequenzielles Versagen der jeweiligen mechanischen Anschläge zu verhindern.

4. Einrichtung nach Anspruch 3, wobei der mindestens eine spiralförmige Schnitt mehrere spiralförmige Schnitte (42) umfasst, wobei (i) jeweilige der mechanischen Anschläge jedes der spiralförmigen Schnitte konfiguriert sind, um gleichzeitig einzugreifen, oder (ii) die mechanischen Anschläge jedes der spiralförmigen Schnitte konfiguriert sind, um gleichzeitig einzugreifen.

5. Einrichtung nach Anspruch 1, wobei der mindestens eine spiralförmige Schnitt mehrere spiralförmige Schnitte (42) umfasst, wobei das Rohr drei der spiralförmigen Schnitte umfasst.

6. Einrichtung nach Anspruch 1, ferner umfassend einen proximalen Koppler (24) mit einem proximalen und distalen Ende (46, 48), wobei das längliche ablenkbare Element ein distales Ende (32) aufweist, das mit dem proximalen Ende des proximalen Kopplers verbunden ist, wobei das Rohr und das distale Ende des proximalen Kopplers komplementäre Bajonettverbindungsmerkmale (50) einschließen, die das distale Ende des proximalen Kopplers mit dem Rohr verbinden.

7. Einrichtung nach Anspruch 1, ferner umfassend einen distalen Koppler (26), wobei das Rohr ein distales Ende (54) aufweist, das Löcher (56) umfasst, die um den Umfang des Rohrs angeordnet sind, wobei das distale Ende des Rohrs über einen Klebstoff, der sich in jeweilige der Löcher erstreckt, mit dem distalen Koppler verbunden ist.

8. Einrichtung nach Anspruch 7, wobei das Rohr eine distale Kante (60) mit Öffnungen (62) darin einschließt, wobei der distale Koppler Vorsprünge (64) einschließt, die zum Eingreifen in die Öffnungen konfiguriert sind, um eine Drehung des distalen Kopplers in Bezug auf das Rohr zu verhindern.

9. Einrichtung nach Anspruch 8, wobei die Öffnungen U-förmige Öffnungen einschließen.

10. Einrichtung nach Anspruch 1, ferner umfassend einen distalen Koppler (26), wobei das Rohr eine distale Kante (60) mit Öffnungen darin einschließt, wobei der distale Koppler Vorsprünge (54) einschließt, die zum Eingreifen in die Öffnungen konfiguriert sind, um eine Drehung des distalen Kopplers in Bezug auf das Rohr zu verhindern, optional wobei die Öffnungen U-förmige Öffnungen einschließen.

11. Einrichtung nach Anspruch 1, wobei die distale Anordnung eine expandierbare distale Anordnung (18) umfasst, optional wobei die expandierbare distale Anordnung einen aufblasbaren Ballon (20) einschließt.

12. Einrichtung nach Anspruch 1, wobei der Kraftsensor eine Sendespule (92) und mindestens eine Empfangsspule (93) einschließt, die an dem Rohr angeordnet sind.

13. Kathetereinrichtung nach Anspruch 1,
wobei sich das Rohr entlang einer Längsachse von einem ersten Ende (54) zu einem zweiten Ende (52) erstreckt, um eine umlaufende Oberfläche zu definieren;
wobei eine Vielzahl von Schlitzen (62) nahe dem ersten Ende auf der umlaufenden Oberfläche des rohrförmigen Elements um die Längsachse angeordnet ist, wobei jeder Schlitz im Allgemeinen parallel zu der Längsachse verläuft;
wobei ein Bajonettelement (50) nahe dem zweiten Ende angeordnet ist, wobei das Bajonettelement drei Arme einschließt, die an dem Umfang des rohrförmigen Elements um die Längsachse angeordnet sind;
wobei der mindestens eine spiralförmige Schnitt mindestens zwei parallele Schnitte (42) umfasst, die sich durch die Oberfläche des im Allgemeinen rohrförmigen Elements über einen Teil des Umfangs des rohrförmigen Elements und entlang der Längsachse erstrecken, sodass die parallelen Schnitte mindestens ein spiralförmiges Federelement definieren, das zwischen dem ersten Ende und dem zweiten Ende nahe dem Umfang des rohrförmigen Elements angeordnet ist; und
wobei die Abweichungen einen mechanischen Anschlag (74, 96) definieren, sodass ein Überbiegen oder Überdehnen des spiralförmigen Federelement verhindert wird.

## Revendications

1. Appareil de cathéter (10), comprenant :
un élément flexible allongé (12) ;
un ensemble distal (14) ;
un capteur de force (28) disposé entre l'élément flexible allongé et l'ensemble distal, et comprenant un ressort (38) comportant un tube (40) avec au moins une découpe hélicoïdale (42) s'étendant autour d'une circonférence du tube, l'au moins une découpe hélicoïdale comportant des déviations (44) s'étendant dans une direction longitudinale du tube, les déviations étant conçues pour empêcher un étirement excessif et un pliage excessif du ressort ;
dans lequel des respectives parmi les déviations des découpes hélicoïdales définissent des butées mécaniques (74, 96) respectives, chaque butée mécanique comportant des surfaces opposées qui sont conçues pour venir en contact l'une avec l'autre pour empêcher un étirement excessif et un pliage excessif du ressort ;
**caractérisé en ce que** des respectives parmi les butées mécaniques comportent des éléments en forme de L (98) respectifs disposés dans des ouvertures en forme de L (100) respectives

2. Appareil selon la revendication 1, dans lequel des respectives parmi les déviations comportent des courbes sigmoïdes opposées (82) respectives.

3. Appareil selon la revendication 1, dans lequel des respectives parmi les butées mécaniques sont conçues pour venir en prise simultanément de sorte qu'une force appliquée sur le ressort est partagée parmi les butées mécaniques respectives pour empêcher une défaillance séquentielle des butées mécaniques respectives.

4. Appareil selon la revendication 3, dans lequel l'au moins une découpe hélicoïdale comprend de multiples découpes hélicoïdales (42), (i) des respectives parmi les butées mécaniques de chacune des découpes hélicoïdales étant conçues pour venir en prise simultanément, ou (ii) les butées mécaniques de chacune des découpes hélicoïdales étant conçues pour venir en prise simultanément.

5. Appareil selon la revendication 1, dans lequel l'au moins une découpe hélicoïdale comprend de multiples découpes hélicoïdales (42), le tube comprenant trois des découpes hélicoïdales.

6. Appareil selon la revendication 1, comprenant en outre un accouplement proximal (24) ayant des extrémités proximale et distale (46, 48), dans lequel l'élément flexible allongé a une extrémité distale (32) reliée à l'extrémité proximale de l'accouplement proximal, le tube et l'extrémité distale de l'accouplement proximal comportant des caractéristiques de liaison à baïonnette complémentaires (50) reliant l'extrémité distale de l'accouplement proximal au tube.

7. Appareil selon la revendication 1, comprenant en outre un accouplement distal (26), dans lequel le tube a une extrémité distale (54) comprenant des trous (56) disposés autour de la circonférence du tube, l'extrémité distale du tube étant reliée à l'accouplement distal par l'intermédiaire d'un adhésif qui s'étend dans des respectifs parmi les trous.

8. Appareil selon la revendication 7, dans lequel le tube comporte un bord distal (60) avec des ouvertures (62) dans celui-ci, l'accouplement distal comportant des parties saillantes (64) conçues pour venir en prise avec les ouvertures pour empêcher une rotation de l'accouplement distal par rapport au tube.

9. Appareil selon la revendication 8, dans lequel les ouvertures comportent des ouvertures en forme de U.

10. Appareil selon la revendication 1, comprenant en outre un accouplement distal (26), dans lequel le tube comporte un bord distal (60) avec des ouvertures dans celui-ci, l'accouplement distal comportant des parties saillantes (54) conçues pour venir en prise avec les ouvertures pour empêcher une rotation de l'accouplement distal par rapport au tube, facultativement dans lequel les ouvertures comportent des ouvertures en forme de U.

11. Appareil selon la revendication 1, dans lequel l'ensemble distal comporte un ensemble distal expansible (18), facultativement dans lequel l'ensemble distal expansible comporte un ballonnet gonflable (20).

12. Appareil selon la revendication 1, dans lequel le capteur de force comporte une bobine de transmission (92) et au moins une bobine de réception (93) disposées sur le tube.

13. Appareil de cathéter selon la revendication 1,
dans lequel le tube s'étend le long d'un axe longitudinal à partir d'une première extrémité (54) jusqu'à une seconde extrémité (52) pour définir une surface circonférentielle ;
dans lequel une pluralité de fentes (62) sont disposées à proximité de la première extrémité sur la surface circonférentielle de l'organe tubulaire autour de l'axe longitudinal, chaque fente étant généralement parallèle à l'axe longitudinal ;
dans lequel un organe à baïonnette (50) est disposé à proximité de la seconde extrémité, l'organe à baïonnette comporte trois bras disposés sur la circonférence de l'organe tubulaire autour de l'axe longitudinal ;
dans lequel l'au moins une découpe hélicoïdale comprend au moins deux découpes parallèles (42) s'étendant à travers la surface de l'organe généralement tubulaire sur une partie de la circonférence de l'organe tubulaire et le long de l'axe longitudinal de sorte que les découpes parallèles définissent au moins un organe de ressort de forme hélicoïdale disposé entre la première extrémité et la seconde extrémité à proximité de la circonférence de l'organe tubulaire ; et
dans lequel les déviations définissent une butée mécanique (74, 96) de sorte que l'organe de ressort hélicoïdal est empêché de plier excessivement ou de s'étirer excessivement.
